# EUROPEAN PATENT SPECIFICATION

(11) **EP 0 410 547 B1**
(45) Date of publication and mention of the grant of the patent: **03.01.1996**
(21) Application number: 90202072.6
(22) Date of filing: 27.07.1990
(51) Int. Cl.: C08F 38/00

(54) **Propargyl aromatic ether polymers**
Polymere aus aromatischen Propargyläthern
Polymères d'éthers de propargyles aromatiques

(30) Priority: 28.07.1989 US 386083; 28.07.1989 US 386079
(43) Date of publication of application: 30.01.1991
(73) Proprietor: SHELL INTERNATIONALE RESEARCH MAATSCHAPPIJ B.V., NL-2596 HR Den Haag (NL)
(72) Inventor: Pigneri, Anthony Michael, Houston, Texas 77036 (US); Bauer, Ronald Sherman, Houston, Texas 77069 (US)

(56) References cited:
- GB-A- 1 021 948
- JOURNAL OF ORGANOMETALLIC CHEMISTRY, vol. 308, no. 2, 8th July 1986, pages C14- C16, Elsevier Sequoia S.A., Lausanne, CH; W. DOUGLAS et al.: "Catalysis of crosslinking of an acetylene-terminated monomer; high activity of nickelocene"
- POLYMER SCIENCE U.S.S.R., vol. 17, no. 5, April 1976, pages 1231-1235, Pergamon Press Ltd, Oxford, GB; L.A. AKOPYAN et al.: "Polymerization of propargyl alcohol (PA) in the presence of nickel chelates"
- CHEMICAL ABSTRACTS, vol. 87, 1977, page 21, abstract no. 6659q, Columbus, Ohio, US; & SU-A-554 269

## Description

The present invention relates to propargyl aromatic ether polymers and their preparation.

Propargyl ethers are a known class of compounds which can be used to prepare certain kinds of polymers. The most common polymers are prepared by oxidative coupling according to Hay et al., Polymer Letters, 8, pp. 97-99 (1970). Other polymers are prepared, e.g., in U.S. patent 4,226,800, by thermal polymerization at high temperatures to give thermoset polymers that are crosslinked. These are unsatisfactory because developments in circuit board technology, such as the use of higher circuit density on the board, surface mounting of components and multilayer circuit boards, demand higher performance from resin systems used in electrical laminates and expose weaknesses in conventional resin laminating systems. Electrical laminates for circuit boards are usually made of a number of alternating copper and resin layers. It is necessary in the process of manufacturing the circuit boards to apply considerable heat to the boards. In this process, if the coefficient of thermal expansion of the resin is not sufficiently low, the resin layers of the laminate may delaminate, or tend to draw away from the copper layers, resulting in poor adhesion of the copper to the resin and breaks in the copper electrical circuit. Another problem resulting from the application of heat to the circuit board is decomposition of the resin resulting in the formation of surface voids and distortion or damage to the copper circuits. To avoid such damage to the board, the resin should have a decomposition temperature higher than about 300 °C.

Other prior art polymers, such as epoxy, bismaleimide, triazine and polyimide resins, do not have the kind of properties desirable in polymers useful to form laminates, mould powders and films for electrical and electronic applications. Accordingly there is a need to develop new polymers for these applications.

The present invention is directed to the use of a propargyl aromatic ether polymer, obtainable by polymerizing a propargyl aromatic ether in the presence of an effective amount of a Group VI or VIII metal complex polymerization catalyst, in a process for the preparation of a laminate, comprising a fibrous filler or reinforcing material.

The J. Organometallic Chem., 308 (1986), pp. C14-16 describes the cross-linking of the dipropargyl ether of bisphenol A (DPE-BPA) via a cyclotrimerization route using certain Group VIII metal complex catalysts. In general terms the curing of acetylene-terminated monomers, like DPE-BPA, is disclosed. However, no physical or chemical properties of the cured products have been indicated. Hence, it is surprising that polymers of propargyl aromatic ethers provide excellent properties to laminates obtained therefrom.

When a Group VI or VIII metal complex polymerization catalyst is used polymers are produced without substantial loss or evolution of void-forming by-products. When cured the propargyl aromatic ether thermoset polymers prepared by the present catalytic polymerization differ from polymers prepared by oxidative coupling and exhibit a lower dielectric constant, a lower coefficient of thermal expansion, a better moisture resistance and a higher glass transition temperature than many other kinds of plastic materials currently in use in electronic and electrical applications, such as epoxy, bismaleimide, triazine and polyimide resins. Such properties enable the new polymers to have the ability to allow for higher electronic signal speeds, survive at higher service temperatures, survive in higher moisture environments, be more compatible with surface mount components, and more easily tolerate printed circuit board processing.

While not being limited to any theory, it is believed, for example, that the cured thermoset polymers of dipropargyl ethers have the formula (1), wherein the curly lines represent polymer chains.

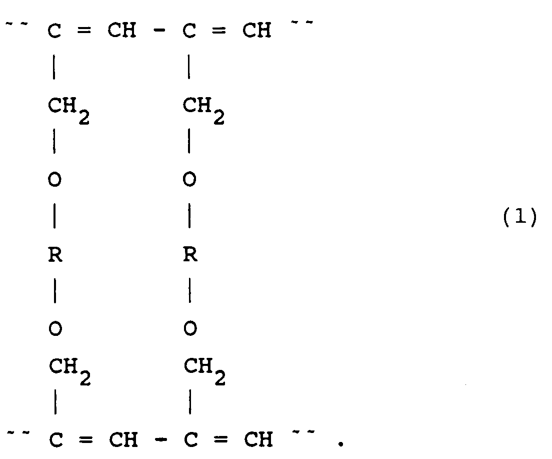

The propargyl aromatic ethers useful in the present inventions are compounds having at least one, preferably at least two, propargyl ether groups ("-OCH₂C≡CH") directly attached to ring carbon atoms of an aromatic (aryl) organic group containing from 6 to 100 carbon atoms. Suitable propargyl aromatic ether monomers include those represented by the formula (2)

R(̵OCH₂ - C ≡ CH)ₙ (2)

wherein n is number of at least from 1 to 10, preferably 2 to 5, and R is an aromatic (aryl) organic group containing up to 100 carbon atoms comprising an aromatic ring or an aromatic ring (a) bonded directly or through a bridging atom or group to or (b) fused to one or more aromatic or cycloaliphatic rings, each aromatic ring of R having from one to all the available ring positions independently substituted by propargyl ether groups. Suitable aromatic organic groups for R include
(i) residues having a valence of at least 2 and derived from aromatic hydrocarbon containing 6 to 16 carbon atoms, such as benzene, naphthalene, anthracene or pyrene;
(ii) organic groups consisting of a plurality of aromatic rings bonded directly or through a bridging atom or group, for example, those expressed by the following formula (3) 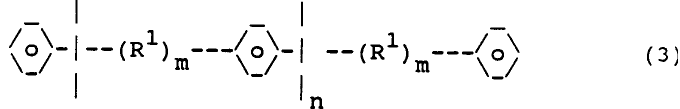 wherein each m is independently zero or 1, n is zero or 1 and each R^{l} is independently a divalent aliphatic or aromatic hydrocarbon or aromatic ether group containing up to 14 carbon atoms, an oxygen atom, a sulphur atom, a carbonyl group, a sulphonyl group, a sulphinyl group, an alkyleneoxyalkylene group containing up to 4 carbon atoms in each alkylene group, an imino group, 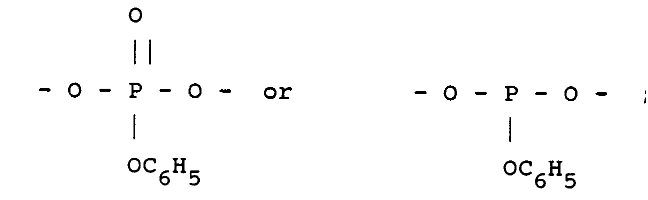 or
(iii) groups derived from novolac-type phenol or resorcinol resins.

These aromatic organic groups R and R¹ can be substituted on their aromatic ring by a substituent which does not participate in the reaction, such as an alkyl group containing 1 to 4 carbon atoms (e.g., methyl or ethyl), an alkoxy group containing 1 to 4 carbon atoms (e.g., methoxy or ethoxy), a halogen atom (e.g., chlorine or bromine), or a nitro groups.

Examples of suitable organic groups of the general formula (2) are those derived from biphenyl, diphenylmethane, α,α-dimethylphenylmethane, diphenyl ether, diphenyl dimethylene ether, diphenyl thioether, diphenyl ketone, diphenylamine, diphenyl sulphoxide, diphenyl sulphone, diphenyl phosphite and triphenyl phosphate.

Propargyl aromatic ethers can be prepared by known methods including reacting a di- or polyhydric phenolic material with a propargyl halide, such as chloride or bromide, in an aqueous alkaline solution, such as aqueous sodium hydroxide solution as described in U.S. Patent 4,226,800. One preferred method is by using propargyl chloride in an aqueous sodium hydroxide and a water-miscible, protic solvent or co-solvent, which is disclosed in EP-A-0369527 (published on 23.05.90). Propargyl aromatic ethers which are preferred in this invention in view of the properties of the final resin obtained are those prepared by reacting a symmetrical, fused ring-free-dihydric phenol, such as bisphenol A or a,a'-bis(4-hydroxyphenyl)-para-di-isopropylbenzene with a propargyl chloride or bromide. This latter novel ether of Formula (4) 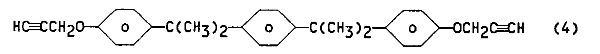 also referred to as dipropargyl aromatic ether of bis(4-hydroxycumyl)benzene (DPE-BHCB), is most preferred, since polymers prepared from this compound have improved adherence to reinforcing materials, and are easily, and in a controlled fashion polymerized to provide a well defined and reliable viscosity. Accordingly, the invention further provides a propargyl aromatic ether polymer obtainable by polymerizing such a propargyl aromatic ether in the presence of an effective amount of a Group VI or VIII metal complex polymerization catalyst. Likewise propargyl aromatic ethers obtained by the reaction of a phenol-formaldehyde precondensate with a propargyl chloride or bromide can advantageously be used.

The catalysts which can be used in the present invention include Group VI (e.g. molybdenum complex, such as molybdenum carbonyl) or Group VIII metal complex catalysts. Preferably, the polymerization catalyst is a nickel, palladium, or platinum complex, such as nickel acetylacetonate, or bis(triphenylphosphine)palladium(II)dichloride; other compounds like bis(1,2-diphenylphosphino)ethane nickel(II)-chloride, tetrakis(triphenylphosphine)palladium(0) may also be used.

Another embodiment of the invention is directed to a laminate comprising a propargyl aromatic ether polymer the latter obtainable by polymerizing a propargyl aromatic ether in the presence of an effective amount of group VI or VIII metal complex polymerization catalyst, and a fibrous filler or reinforcing material.

Another embodiment of the invention is directed to a process for preparing a laminate comprising the following steps:
i) combining a propargyl aromatic ether with an effective amount of the Group VI or VIII metal complex polymerization catalyst and a fibrous filler or reinforcing material;
ii) forming a mass thereof, and
iii) compressing and hardening the mass at elevated temperatures.

The polymerization is suitably carried out in the presence of a solvent. The solvent is generally an oxygenated solvent such as a ketone, alcohol, glycol, glycol ether, glycol ether acetate, THF and dimethyl formamide. Hydrocarbon or chlorinated hydrocarbon solvents might also be used.

The fibrous fillers and reinforcing materials used in the present invention are conventional kinds of materials known in the art. Suitable materals include, but are not limited to, glass, carbon, "KEVLAR®" (trademark), boron, calcium carbonate, talc, alumina and asbestos and where appropriate, the fibre forms thereof. The preferred fibrous reinforcing materials are selected from the group consisting of glass fibres, quartz fibres, carbon fibre, boron fibres, TEFLON®" (trademark) fibres (polytetrafluoroethylene) and "KEVLAR®" (trademark) fibres, with woven or continuous glass fibres or carbon fibres being preferred. The fibrous or reinforcing material is present in the composition in an amount effective to impart increased strength to the composition for the intended purpose, generally from 20 to 95 weight per cent, usually from 35 to 80 weight per cent, based on the weight of the total composition. The laminates of the invention can optionally include one or more layers of a different materials and in electrical laminates this includes one or more layers of a conductive material such as copper.

The propargyl aromatic ether composition can be applied to the fibrous or reinforcing material neat or from a solution in a suitable solvent of the type disclosed above.

The curable composition of this invention can be used in any desired form such as solid, solution or dispersion. These curable compositions include those mixed in solvent or in the absence of a solvent to form the compositions of this invention. For example, the mixing procedure comprises mixing solutions of the monomer and polymerization catalyst in a suitable inert organic solvent, such as for example, ketones such as methyl ethyl ketone, chlorinated hydrocarbons such as methylene chloride and ethers, and homogenizing the resulting mixed solution at room temperature or at an elevated temperature below the boiling point of the solvents to form a composition in the form of a solution. When homogenizing these solutions at room temperature or at an elevated temperature, some reactions may take place between the constituent elements. So long as the resins components are maintained in the state of solution without gelling, such reactions do not particularly affect the operability of the resulting composition in, for example, a bonding, coating, laminating or moulding operation.

The curable resin compositions of this invention can be used in the above solution form as adhesives, paints vehicles, moulding materials to be impregnated in substrates, or laminating materials. In this case, the concentration of the resin solid in the solution is determined so that the optimum operability can be obtained according to the desired utility.

The resin compositions of this invention can be used for various purposes in the form of dried powder, pellets, resin-impregnated product or compound. For example, compositions with the individual components uniformly mixed can be obtained by uniformly mixing the resin components in solution, and then removing the solvents from the homogeneous solution at reduced pressure or at an elevated temperature. Alternatively, solid components are kneaded at room temperature or at an elevated temperature to form a homogenized resin composition.

A variety of additives may be added to the composition to impart specific properties of the compositions in their monomeric or polymeric state provided that they do not impair the essential properties of the resulting resin. Examples of the additives include natural or synthetic resins, fibrous reinforcement, fillers, pigments, dyestuffs, thickening agents, wetting agents, lubricants, (flame-)retardants and accelerators.

The resin composition of this invention can also contain a white pigment such a titanium dioxide, a coloured pigment such as yellow lead, carbon black, iron black, molybdenum red, Prussian blue, ultramarine, cadmium yellow or cadmium red, and other various organic and inorganic dyes and pigments in order to colour the compositions. In addition to the above coloured pigments, the resin compositions can also contain a rust-proofing pigment such as zinc chromate, red lead, red iron oxide, zinc flower or strontium chromate, an anti-sag agent such as aluminium stearate, a dispersing agent, a thickener, a coat modifier, a body pigment or a fire retardant, which are known additives for paints.

The compositions of this invention are cured by heating after applying it to a substrate as a coating or adhesive layer, or after moulding or laminating in the form of powder, pellet or as impregnated in a substrate. The curing conditions of the curable composition of this invention depend on the proportion of components constituting the composition and the nature of the components employed. In general, the composition of this invention may be cured by heating it at temperature within the range of 0-300 °C, preferably 100-250 °C, although differing according to the presence of a catalyst or curing agent or its amount, or the types of the components in the composition. The time required for heating is generally 30 seconds to 10 hours, although considerably differing according to whether the resin composition is used as a thin coating or as moulded articles of relatively large thickness or as laminates or as matrix resins for fibre reinforced composites, particularly for electrical and electronic applications, e.g., when applied to an electrically nonconductive material, such as glass, subsequently cured and then coated with a conductive material such as copper. Layers of such coated materials can be used to make laminates. When the resin composition of this invention is used for producing moulded articles, laminated articles or bonded structures, the curing is desirably effected under pressure. Generally, this pressure is from 0.49 to 19.73 MPa (gauge) 5 to 200 Kg/cm.

The composition of this invention cures rapidly, even under mild conditions, so is especially suitable when quantity production and ease of workability are desired. The cured resin made from the composition not only has excellent adhesive force, bond strength, heat resistance, and electric properties, but also is excellent in mechanical properties and resistance to impact, chemicals and moisture The composition of this invention has a variety of uses as a coating material for rust prevention, flame resistance, flame retardance; as electrical insulating varnish; as adhesive; in laminates to be used for furnitures, building materials, sheathing materials, electrical insulating materials; and in a variety of mouldings.

A more complete understanding of the present invention is provided by referring to the following illustrative embodiments, which are not intended to limit the invention in any way.

### Embodiment 1

The dipropargyl ether (DPE) of bis(hydroxycumyl)benzene (BCHB) was prepared by treating 200 g of BCHB with 168 g of propargyl chloride added over about 1/2 hour at 57 °C in the presence of (a) sodium hydroxide in a molar ratio sodium hydroxide to phenol of 1.25 and (b) a reaction medium comprising 669 g of isopropyl alcohol (IPA) and 2050 g of water at 65-68 °C. The reaction was continued for about 25 hours while maintaining the pH ≥ 11. The reaction product was cooled to room temperature, where the product crystallized out of solution. This product was washed twice with isopropyl alcohol (IPA) and then with water until a constant pH of wash water was obtained. The product crystals were then blown dry to obtain the desired product in 67% yield (179.8 g) Product was then recrystallized by dissolving into boiling IPA, filtering to remove any residues, and allowing to cool to room temperature. The filtrate, now cloudy and containing precipitate, was further cooled to 0 °C overnight. Then, the cold mixture was filtered, the solids washed with IPA, and dried in a vacuum oven overnight, at room temperature. A vacuum of 0.85 bar (25 in Hg) was maintained by bleeding nitrogen into the oven. Final realized yield was 58.7 %wt, or 157.4 grams.

### Embodiment 2

The effectiveness of various catalysts for polymerizing dipropargyl ether of bisphenol A (DPE-BPA) was determined by forming a mixture of 1 g of the ether with 0.01 g of various catalysts shown in Table 1 below and measuring the exotherm temperature in °C.

**Table 1**

| Dipropargyl Ether of Bisphenol A Polymerization | |
|---|---|
| Catalyst | Temperature °C |
| bis(triphenylphosphine)palladiumdichloride | 209.2 |
| tetrakis(triphenylphosphine)palladium(0) | 212 |
| molybdenum carbonyl | 164.9; 290 |
| nickel acetylacetonate | 148.3 |
| bis(1,2-diphenylphosphino)ethanenickeldichloride | 176.9 |
| no catalyst | 301.5 |

### Embodiment 3

Prepregs were prepared from DPE-BPA and DPE-BHCB resin compositions as follows:

**Table 2**

| Dipropargyl Ether Prepreg Manufacture from Solvent Borne Resin Varnish | | |
|---|---|---|
| Property | Value | |
| Resin | DPE-BPA | DPE-BHCB |

| Varnish | | |
|---|---|---|
| Formulation, %wt | | |
| DPE-BPA | 49.9 | - |
| DPE-BHCB | - | 49.9 |
| Dimethyl formamide | 17.3 | 17.3 |
| Toluene | 16.4 | 16.4 |
| Acetone | 16.4 | 16.4 |
| Bis(triphenylphoshine)-palladiumdichloride, phr (ppm Pd) | 0.26 (400) | 0.40 (600) |
| Gel Time, Seconds @ 171 °C | 225 | 245 |

| Prepreg | | |
|---|---|---|
| Glass Style | 7628 | 7628 |
| Processing Conditions | | |
| Oven Time, Minutes | 4.0 | 4.0 |
| Oven Temperature, °C | 163 | 163 |
| Resin Content, %wt | 23 | 28 |

### Embodiment 4

Prepregs and laminates were prepared from DPE-BPA and DPE-BHCB resin compositions as follows:

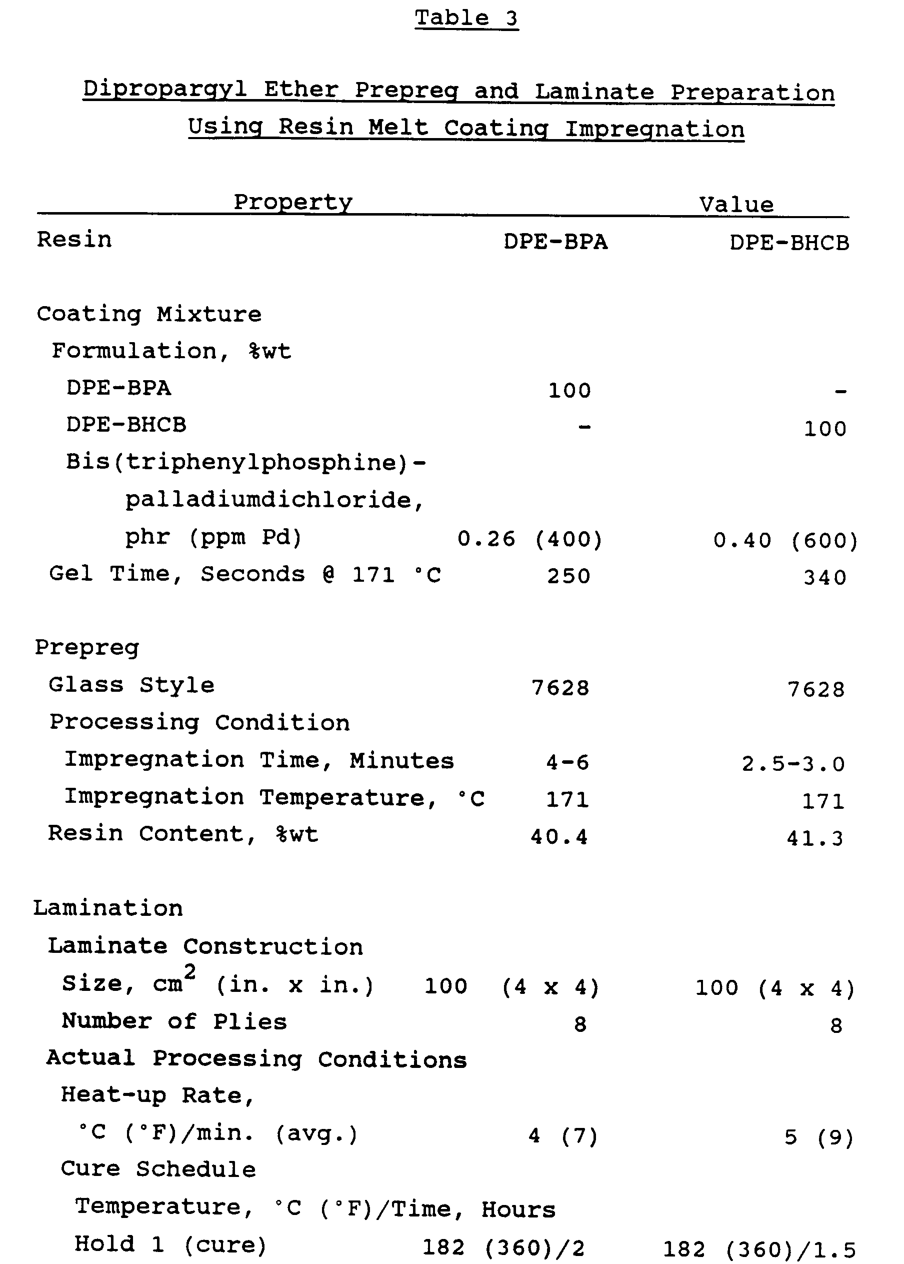

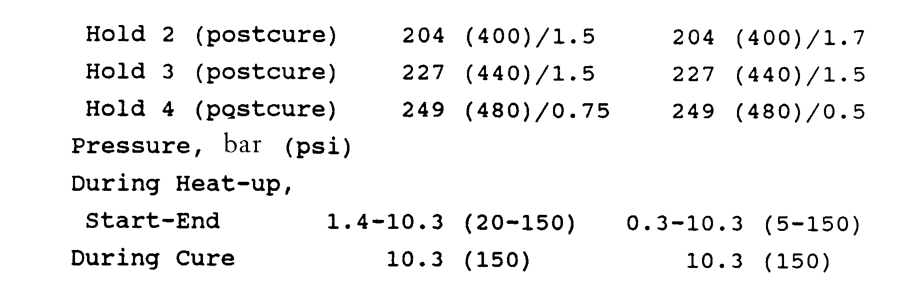

The properties of the resulting laminates are set forth in Table 4 as compared to a standard brominated epxoy resin laminate.

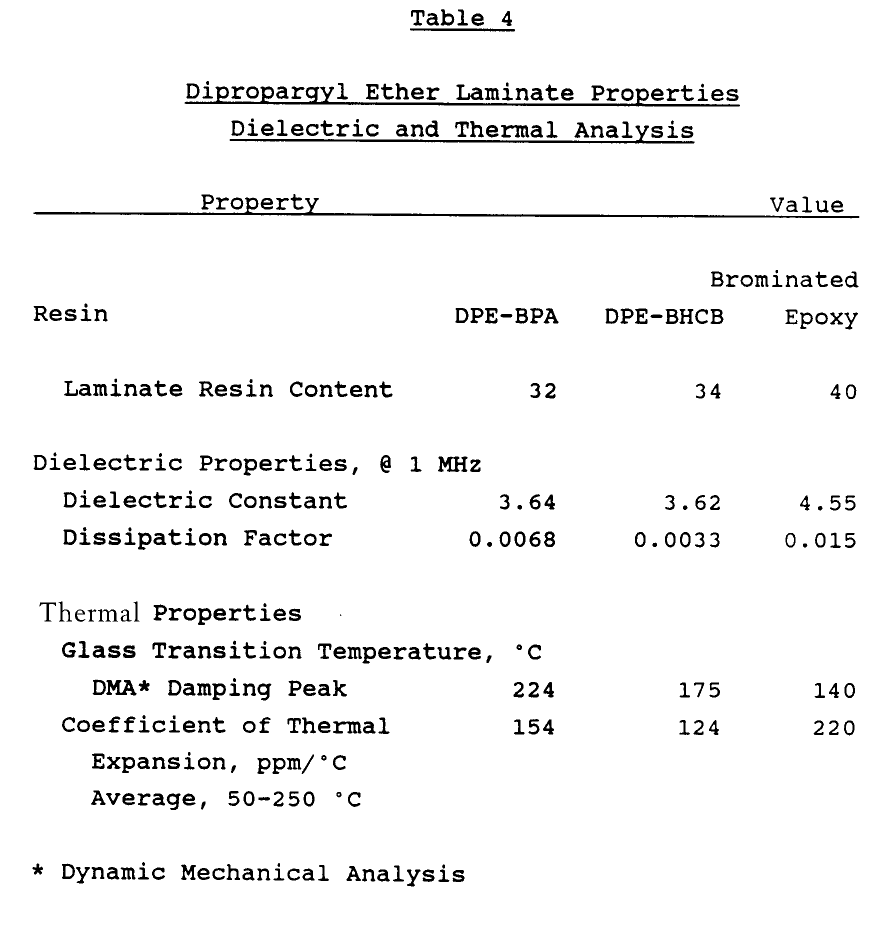

As shown in Table 4 above, the dipropargyl ether resin laminates do have preferred lower dielectric constants and dissipation factors than the standard brominated epoxy resin laminate even at lower resin content. The Tg's of the dipropargyl ether-based laminates were also much higher than for the standard brominated epoxy resin-based laminate. The coefficient of thermal expansion of the dipropargyl ether-based laminate is also lower than for the standard brominated epoxy-based laminate.

## Claims

1. Use of a propargyl aromatic ether polymer, obtainable by polymerizing a propargyl aromatic ether in the presence of an effective amount of a Group VI or VIII metal complex polymerization catalyst, in a process for the preparation of a laminate, comprising a fibrous filler or reinforcing material.

2. Use as claimed in claim 1, wherein the polymerization catalyst is a nickel, palladium or platinum complex polymerization catalyst.

3. Use as claimed in claim 2, wherein the polymerization catalyst is nickel acetylacetonate or bis(triphenylphosphine)palladium(II)dichloride.

4. Use as claimed in any one of claims 1 to 3, wherein the propargyl aromatic ether is a compound of Formula (4)

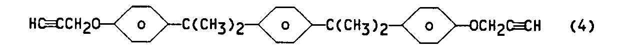

5. Use as claimed in any one of claims 1 to 4, which comprises polymerizing the propargyl aromatic ether in the presence of a solvent.

6. Use as claimed in claim 5, wherein the solvent is a mixture of toluene, dimethylformamide and acetone.

7. A process for the preparation of a laminate comprising the following steps:
i) combining a propargyl aromatic ether with an effective amount of the Group VI or VIII metal complex polymerization catalyst and a fibrous filler or reinforcing material;
ii) forming a mass thereof, and
iii) compressing and hardening the mass at elevated temperatures.

8. A laminate comprising a propargyl aromatic ether polymer the latter obtainable by polymerizing a propargyl aromatic ether in the presence of an effective amount of group VI or VIII metal complex polymerization catalyst, and a fibrous filler or reinforcing material.

9. A propargyl aromatic ether of Formula (4)

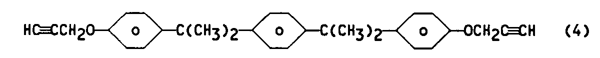

10. A propargyl aromatic ether polymer obtainable by polymerizing a propargyl aromatic ether as claimed in claim 9 in the presence of an effective amount of a Group VI or VIII metal complex polymerization catalyst.

## Patentansprüche

1. Verwendung eines Polymers aus einem aromatischen Propargylether, welches durch Polymerisation eines aromatischen Propargylethers in Gegenwart einer wirksamen Menge eines aus einem Gruppe-VI- oder einem Gruppe-VIII-Metall-Komplex bestehenden Polymerisationskatalysators erhältlich ist, bei einem Verfahren zur Herstellung eines Schichtstoffs mit einem faserförmigen Füllstoff oder Verstärkungsmaterial.

2. Verwendung nach Anspruch 1, bei der es sich bei dem Polymerisationskatalysator um einen aus einem Nickel-, Palladium- oder Platinkomplex bestehenden Polymerisationskatalysator handelt.

3. Verwendung nach Anspruch 2, bei der es sich bei dem Polymerisationskatalysator um Nickelacetylacetonat oder Bis(triphenylphosphin)palladium(II)-dichlorid handelt.

4. Verwendung nach einem der Ansprüche 1 bis 3, bei der es sich bei dem aromatischen Propargylether um eine Verbindung der Formel (4) 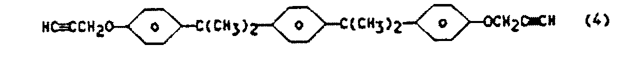 handelt.

5. Verwendung nach einem der Ansprüche 1 bis 4, bei der man den aromatischen Propargylether in Gegenwart eines Lösungsmittels polymerisiert.

6. Verwendung nach Anspruch 5, bei der es sich bei dem Lösungsmittel um ein Gemisch aus Toluol, Dimethylformamid und Aceton handelt.

7. Verfahren zur Herstellung eines Schichtstoffs, mit den folgenden Verfahrensschritten:
i) Kombination eines aromatischen Propargylethers mit einer wirksamen Menge des aus einem Gruppe-VI-oder einem Gruppe-VIII-Metall-Komplex bestehenden Polymerisationskatalysators und einem faserförmigen Füllstoff oder Verstärkungsmaterial,
ii) Bildung einer Masse daraus, und
iii) Verdichten und Härten der Masse bei erhöhter Temperatur.

8. Schichtstoff, bestehend aus einem Polymer aus einem aromatischen Propargylether, welches man durch Polymerisation eines aromatischen Propargylethers in Gegenwart einer wirksamen Menge eines aus einem Gruppe-VI- oder einem Gruppe-VIII-Metall-Komplex bestehenden Polymerisationskatalysators erhält, und einem faserförmigen Füllstoff oder Verstärkungsmaterial.

9. Aromatischer Propargylether der Formel (4)

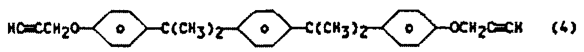

10. Polymer aus einem aromatischen Propargylether, erhältlich durch Polymerisation eines aromatischen Propargylethers nach Anspruch 9 in Gegenwart einer wirksamen Menge eines aus einem Gruppe-VI- oder einem Gruppe-VIII-Metall-Komplex bestehenden Polymerisationskatalysators.

## Revendications

1. Utilisation d'un polymère d'éther propargylaromatique, que l'on peut obtenir par la polymérisation d'un éther propargylaromatique en présence d'une quanttité efficace d'un catalyseur de polymérisation à < plexe d'un métal du groupe VI ou du groupe VIII dans un procédé de fabrication d'un lamifié comprenant une matière de renforcement ou une charge fibreuse.

2. Utilisation suivant la revendication 1, caractérisée en ce que le catalyseur de polymérisation es catalyseur de polymérisation à complexe de nickel, palladium ou de platine.

3. Utilisation suivant la revendication 2, caractérisée en ce que le catalyseur de polymérisation l'acétylacétonate de nickel ou le dichlorure de 1 (triphénylphosphine)palladium(II).

4. Utilisation suivant l'une quelconque revendications 1 à 3, caractérisée en ce que l'éther propargylaromatique est un composé de la formule (4)

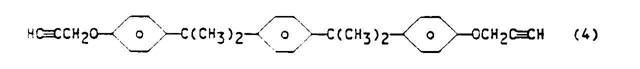

5. Utilisation suivant l'une quelconque des revendications 1 à 4, caractérisée en ce qu'elle comprend la polymérisation de l'éther propargylaromatique en présence d'un solvant.

6. Utilisation suivant la revendication 5, caractérisée en ce que le solvant est constitué par un mélange de toluène, de diméthylformamide et d'acétone.

7. Procédé de fabrication d'un lamifié comprenant les étapes suivantes :
i) combinaison d'un éther propargylaromatique à une quantité efficace d'un catalyseur de polymérisation à complexe d'un métal du groupe VIII ou VI et une matière de renforcement ou une charge fibreuse,
ii) formation d'une masse à partir de cette combinaison et
iii) compression et durcissement de la masse à des températures élevées.

8. Stratifié comprenant un polymère d'éther propargylaromatique, ce dernier pouvant être obtenu par la polymérisation d'un éther propargylaromatique en présence d'une quantité efficace d'un catalyseur de polymérisation à complexe d'un métal du groupe VI ou VIII et d'une matière de renforcement ou d'une charge fibreuse.

9. Ether propargylaromatique de la formule (4)

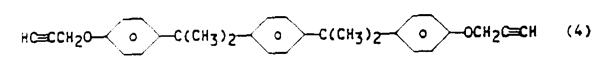

10. Polymère d'éther propargylaromatique que l'on peut obtenir par la polymérisation d'un éther propargylaromatique suivant la revendication 9 en présence d'une quantité efficace d'un catalyseur de polymérisation à complexe d'un métal du groupe VI ou VIII.
